# EUROPÄISCHE PATENTSCHRIFT

(11) **EP 4 144 340 B1**
(45) Veröffentlichungstag und Bekanntmachung des Hinweises auf die Patenterteilung: **25.03.2026**
(21) Anmeldenummer: 22185395.5
(22) Anmeldetag: 18.07.2022
(51) Int. Cl.: A61K 8/44, A61K 8/60, A61K 8/73, A61Q 5/00, A61Q 5/02

(54) **SHAMPOOZUBEREITUNG MIT GUARHYDROXYPROPYLTRIMONIUMCHLORIDEN**
SHAMPOO PREPARATION WITH GUAR HYDROXYPROPYLTRIMONIUM CHLORIDES
PRÉPARATION DE SHAMPOOING CONTENANT DES CHLORURES DE GUARHYDROXYPROPYLTRIMONIUM

(30) Priorität: 06.09.2021 DE 102021209763
(43) Veröffentlichungstag der Anmeldung: 08.03.2023
(73) Patentinhaber: Beiersdorf AG, 22529 Hamburg (DE)
(72) Erfinder: Reiter, Katharina, 21357 Barum (DE); Stoltze, Pia, Hamburg 22455 (DE); Nemnich, Julia, 22589 Hamburg (DE)

(56) Entgegenhaltungen:
- EP-A1- 1 366 738
- EP-A1- 1 366 739
- WO-A1-2013/011122
- CA-C- 2 716 438
- US-A1- 2004 223 991
- US-A1- 2018 325 791

## Beschreibung

Kosmetische Produkte dienen im Allgemeinen nicht nur dazu, schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut und Haar eingesetzt.

Bei der Körperpflege des Menschen spielt die Haarwäsche eine zentrale Rolle. Die Reinigung der Haare und der Kopfhaut von körpereigenem Fett, Hautabschilferungen, Schmutz und Gerüchen entspricht einem Grundbedürfnis des Menschen.

Zur Befriedigung dieses Bedürfnisses stand dem Menschen bis ins 20.Jahrhundert hinein allein Seife zur Verfügung, die aufgrund ihres alkalischen pH-Wertes für die Kopfhaut und die Augenschleimhäute wenig verträglich war und häufig Ablagerungen von Kalkseifen im Haar zurückließ. In den dreißiger Jahren des 20.Jahrhunderts erblickte das erste alkylsulfathaltige Shampoo das Licht der Welt. Seit Mitte der sechziger Jahre erobern Alkylethersulfate und andere Tenside den Shampoomarkt. Mit ihnen können die Nachteile von seifenhaltigen Zubereitungen vermieden werden. Heutzutage müssen moderne Shampoos das Haar nicht nur reinigen und gut verträglich sein. Vielmehr sollen sie das Haar auch pflegen und seine Frisierbarkeit und optische Attraktivität erhöhen.

Zur Befriedigung dieses Bedürfnisses stand dem Menschen bis ins 20.Jahrhundert hinein allein Seife zur Verfügung, die aufgrund ihres alkalischen pH-Wertes für die Kopfhaut und die Augenschleimhäute wenig verträglich war und häufig Ablagerungen von Kalkseifen im Haar zurückließ. In den dreißiger Jahren des 20.Jahrhunderts erblickte das erste alkylsulfathaltige Shampoo das Licht der Welt. Seit Mitte der sechziger Jahre erobern Alkylethersulfate und andere Tenside den Shampoomarkt. Mit ihnen können die Nachteile von seifenhaltigen Zubereitungen vermieden werden. Heutzutage müssen moderne Shampoos das Haar nicht nur reinigen und gut verträglich sein. Vielmehr sollen sie das Haar auch pflegen und seine Frisierbarkeit und optische Attraktivität erhöhen.

Haarshampoos enthalten eine Vielzahl unterschiedlicher Komponenten, um den einzelnen Anforderungen an das Produkt gerecht zu werden:
Die Reinigungskraft der Shampoos wird bewirkt durch die Anwesenheit von anionischen, amphoteren und nichtionischen Tensiden als oberflächenaktive Verbindungen in den Zubereitungen. Tenside sorgen darüber hinaus für das Schaumvermögen der Haarreinigungsmittel. Wichtig bei der Auswahl der Tenside ist darüber hinaus ihre Unempfindlichkeit gegenüber der Wasserhärte, ihre biologische Abbaubarkeit, ihre Verträglichkeit mit anderen Komponenten der Zubereitung sowie ihr Preis. Ein viel verwendetes Shampootensid ist beispielsweise Alkylethersulfat.

Darüber hinaus enthalten Shampoos eine Reihe von Konsistenzregulatoren, die der Zubereitung die gewünschte Viskosität verleihen. Diese Verdicker bewirken eine Vergrößerung der Tensidmicellen bzw. eine Quellung der Wasserphase der Zubereitung. Verdicker können aus chemisch sehr unterschiedlichen Stoffklassen gewählt werden. So werden u.a. Elektrolyte (z.B. Natriumchlorid), Alkanolamide (z.B. Fettsäure-Monoethanolamide), niedrig ethoxylierte Fettalkohole (z.B. Diethylenglycol-monolaurylether), hochethoxylierte Ether, Ester und Diester sowie polymere Verdicker eingesetzt. Zu den polymeren Verdickern zählen beispielsweise Celluloseether. Darüber hinaus finden auch Polyacrylate und Hydrokolloide als Verdicker Verwendung. Polymere Verdicker haben den großen Vorteil, dass die durch sie erzeugte Viskosität weitgehend temperaturunabhängig ist.

Neben Parfüm- und Farbstoffen sowie einer Reihe von Verbindungen, welche die Haltbarkeit der Zubereitungen erhöhen, werden in jüngerer Zeit den Haarshampoos unterschiedliche Arten von Wirkstoffen zugefügt. Hierzu zählen neben UV-Absorbern, Vitaminen oder Pflanzenextrakten auch sogenannte Haarkonditionierer (engl. conditioner), welche das Haar pflegen und seine Kämmbarkeit und seinen Griff verbessern sowie seinen Glanz erhöhen. Konditionierer ziehen, im Gegensatz zu den meisten anderen Bestandteilen von Shampoos, auf das Haar auf und verbleiben dort nach dem Spülen. Sie lagern sich aufgrund ihres Molekülaufbaus an die Schadstellen der Cuticula des Haares und glätten das Haar. Dadurch wird das Haar weniger rau und spröde, die Frisur bekommt deutlich mehr Glanz und läßt sich leichter kämmen. Auch wird das Haar weniger empfindlich für eine elektrostatische Aufladung. Die wichtigsten haarkonditionierenden Substanzen, in der Regel kationische Polymere, stellen die polymeren quaternären Ammoniumverbindungen dar. Auch können kationische Cellulosederivate und Polysaccharide eingesetzt werden. Weiterhin werden auch Silikonverbindungen zur Konditionierung eingesetzt.

Ein Nachteil am Stande der Technik besteht in dem Umstand, dass durch häufiges Waschen oder chemische Behandlung (Bleichen, Färben) das Haar stark geschädigt wird. Insbesondere führt die Entfernung/Zerstörung von natürlich im Haar vorkommenden Fettkomponenten zu einem Verlust an Glätte, Geschmeidigkeit und Glanz. Das Haar erscheint dann trocken und spröde. Um diese Schäden ausgleichen zu können, werden den Haarpflegeprodukten in der Regel große Mengen an kationischem Polyquaternium-10 als Haarkonditionierer zugesetzt. Um den Fettverlust auszugleichen, werden derartigen Produkten häufig Silikonverbindungen oder weitere hydrophobe Konditionierer zugesetzt. Diese nach dem Stande der Technik hergestellten Produkte haben jedoch eine Reihe von Nachteilen:
So ist der Einsatz von Polymeren, welche Homo- oder Copolymerisation mit Acrylsäure oder Methacrylsäure, sowie von chemisch modifizierten kationischen Polymeren, wie Polyquaternium-10, von Verbrauchern nicht mehr erwünscht, da die biologische Abbauarbeit nicht abschließend geklärt ist. Entsprechend besteht ein Bedarf an Produkten, die nicht die genannten Inhaltstoffe enthalten. Weiterhin ist der Einsatz von Silikonverbindungen in Shampoos aufgrund ungeklärter Verträglichkeiten, unnötiger Beschwerung der Haare und schlechter Bioabbaubarkeit nicht mehr erwünscht. Bei Verzicht auf diese Komponenten kommt es unweigerlich zu schlechteren Produkteigenschaften. Jedoch erwarten die Verbraucher, trotz Verzichts, insbesondere von Polyquaternium-10, von den käuflich erhältlichen Shampoo-Produkten, dass eine vergleichbare Produktperformance erreicht wird.
So ist es unter anderem wünschenswert vergleichbare oder auch verbesserte
   - Kämmbarkeit von trockenen und nassen Haaren;
   - Gleitvermögen nach Auftragen auf nasse Haare;
   - Ausspülbarkeit der Produkte aus den Haaren;
   - Beschwerung (bzw. Ausbleiben eben dieser);
   - Schaumeigenschaften (schnelles und leichtes Anschäumverhalten, Reichhaltigkeit) und/oder
   - Gleitvermögen nach dem Ausspülen
zu erreichen.

Im Stand der Technik sind verschiedene Möglichkeiten beschrieben, wie diese Parameter erreicht werden können. Jedoch bedarf es oftmals größerer Mengen an biologisch abbaubaren Inhaltstoffe, um eine geeignete Produktperformance gemäß dem vorstehend beschriebenen Parameter zu erreichen. Dieses erhöht die Produktionskosten für die Hersteller, so dass Produkte nur zu einem erhöhten Verkaufspreis abgegeben werden können. Dieses ist jedoch nicht im Sinne der Unternehmen und der Verbraucher.

Folglich bedarf es neuer Ansätze, bei zumindest einige der Parameter mit möglichst geringen Einsatzkonzentrationen von insbesondere biologisch abbaubaren kationischen Polymeren erreicht werden können.

Dem Fachmann ist der Einsatz von Guarhydroxypropyltrimoniumchlorid in Shampoozubereitungen unter anderem aus EP1366742, EP1366738 oder EP1366739 bekannt.

Guarhydroxypropyltrimoniumchlorid ist ein Polymer, welches aus Guarmehl gewonnen werden kann. Abhängig von der gewollten Masse und Ladungsdichte kann das Syntheseverfahren angepasst werden. Mögliche Syntheseverfahren und die daraus erhaltenen Polymere mit gewünschter Masse und Ladungsdichte sind u.a. aus WO 2013/011122 A1 oder US4663159A bekannt.

Nachfolgend ist eine kurze Auswahl an Guarhydroxypropyltrimoniumchlorid Polymeren mit mittlerer Masse und Ladungsdichte angegeben:

**Tab.1**

| Handelsname | Mittlere Masse in Da | Ladungsdichte in meq/g |
|---|---|---|
| Jaguar^{®} C-500 (Rhodia)³ | 389 000 | 0,72 |
| Jaquar^{®} C-17 (Rhodia)³ | 2 000 000 | 1,04 |
| Jaquar^{®} C 13S (Rhodia)¹ | 2 200 000 | 0,8 |
| Jaquar^{®} C-14s (Rhodia)³ | 2 000 000 | 0,72 |
| Jaguar^{®} Optima (Rhodia)² | 500 000 | 1,3 |
| Jaguar^{®} Excel (Solvay)² | 1 500 000 | 0,7 |
| Hi-Care 1000 (Rhodia)¹ | 600 000 | 0,7 |
| N-Hance 3270 (ASI/Ashland)¹ | 425 000 | 0,7 |
| N-Hance 3196 (ASI/Ashland)¹ | 1 100 000 | 0,8 |
| AquaCat CG518 (ASI/Ashland)¹ | 50 000 | 0,9 |
| Polymer 1 from WO 2013/011122 A1 | 333 000 | 1,25 |
| Polymer 2 from WO 2013/011122 A1 | 1 190 000 | 1,37 |
| Polymer 3 from WO 2013/011122 A1 | 305 000 | 1,17 |
| Polymer 4 from WO 2013/011122 A1 | 368 000 | 1,37 |

| | | |
|---|---|---|
| ¹Bekannt aus FP 2999455 A1 ²Bekannt aus US20180311135 A1 ³Bekannt aus WO 2013/011122 A1 | | |

Der Begriff "Ladungsdichte", wie er hier verwendet wird, bezieht sich auf das Verhältnis positiver Ladungen an einer Monomereinheit, aus der ein Polymer besteht, zum Molekulargewicht der Monomereinheit. Die Ladungsdichte wird in meq/g (Milli-Äquivalente pro Gramm) angeben. Die Ladungsdichte multipliziert mit dem Molekulargewicht des Polymers bestimmt die Anzahl der positiv geladenen Stellen an einer gegebenen Polymerkette.

Der Begriff "kationische Gruppen", wie er hier verwendet wird, bezieht sich auf positiv geladene Gruppen und auf teilweise geladene Gruppen. Der Ausdruck "teilweise geladene Gruppen", wie er hier verwendet wird, bezeichnet Gruppen, die in Abhängigkeit vom pH-Wert der Formulierung positiv geladen werden können. Solche Gruppen können auch als "potentiell kationische Gruppen" bezeichnet werden. Wie hier verwendet, bedeutet der Begriff "kationisch" zumindest teilweise kationisch. So umfassen die Begriffe "kationisierende Mittel", "kationische Gruppen" und "kationische Einheiten" Ammonium (die eine positive Ladung aufweisen), aber auch primäre, sekundäre und tertiäre Amine und deren Vorläufer (die zu positiv geladenen Verbindungen führen können).

Messtechnisch kann für kationische Guars die Ladungsdichte unter Verwendung einer Standardelementaranalyse des prozentualen Stickstoffs gemessen werden, welche dem Fachmann bekannt ist. Bei den kationischen Copolymeren hängt die Ladungsdichte von den bei der Synthese verwendeten Monomeren ab. Dem Fachmann bekannte Standard-NMR-Techniken werden verwendet, um dieses Verhältnis von kationischen und nichtionischen Monomeren in dem Polymer zu bestätigen.

Trotz, dass eine Vielzahl an Dokumenten den Einsatz von Guarhydroxypropyltrimoniumchlorid in Shampoozubereitungen dokumentiert, konnte keines der Dokumente den Fachmann zum Gegenstand der vorliegenden Erfindung führen.

Überraschend konnte nun eine Shampoozubereitung bereitgestellt werden, welche die genannten Eigenschaften von Polyquaternium-10 haltigen Zubereitungen aufweist, ohne dass dabei höhere kationische Polymerkonzentrationen einzusetzen. Überraschend gelöst wurde die Aufgabe durch den Gegenstand der vorliegenden Erfindung.

Gegenstand der vorliegenden Erfindung ist eine Shampoozubereitung enthaltend
a) mindestens ein erstes Guarhydroxypropyltrimoniumchlorid, welches ein mittleres Molekulargewicht von mehr als 2000000 bis 2500000 Dalton aufweist und eine Ladungsdichte von 0,5 bis 1,0 meq/g besitzt,
b) mindestens ein zweites Guarhydroxypropyltrimoniumchlorid, welches ein mittleres Molekulargewicht von 400000 bis weniger als 2000000 Dalton aufweist und eine Ladungsdichte von 0,5 bis 1,0 meq/g besitzt,
c) ein oder mehrere Tenside
wobei das mittlere Molekulargewicht mittels Lichtstreuung ermittelt wird.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der Zubereitung. Sollten nachfolgend Verhältnisse von Komponenten/Substanzen/Stoffgruppen offenbart werden, so beziehen sich diese Verhältnisse auf Gewichtsverhältnisse der genannten Komponenten/ Substanzen/Stoffgruppen.

Werden nachfolgend Gewichtsprozentbereiche für die Bestandteile der Zubereitung angegeben, so umfasst die Offenbarung der vorliegenden Anmeldung ebenfalls alle Einzelwerte in Schritten von 0,1 Gew.-% innerhalb dieser Gewichtsprozentbereiche.

Die Formulierungen "erfindungsgemäß", "erfindungsgemäß vorteilhaft", "vorteilhaft im Sinne der vorliegenden Erfindung" etc. beziehen sich im Rahmen der vorliegenden Offenbarung immer sowohl auf die erfindungsgemäße Zubereitung sowie die erfindungsgemäße Verwendung und das Verfahren.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Wird der Begriff Haut verwendet, so bezieht dieser sich bevorzugt auf die menschliche Haut. Alle nachfolgend genannten Substanzen werden anhand Ihre INCI Deklaration oder mittels eindeutiger chemischer Namen benannt.

Unter Emulgatoren werden alle Substanzen verstanden, welche im International Cosmetic Ingredient Dictionary and Handbook, Thirteenth Edition 2010, (ISBN 1-882621-47-6) unter der Bezeichnung "emulsifying agent" geführt werden. Unter Tensiden werden alle Substanzen verstanden, welche im International Cosmetic Ingredient Dictionary and Handbook, Thirteenth Edition 2010, (ISBN 1-882621-47-6) unter der Bezeichnung " surfactant " geführt werden.

Werden in dieser Offenbarung Viskositätswerte angegeben, so beziehen sich alle Werte auf eine Messung bei 25°C in einer 150 ml Weithalsflasche (VWR Nr.: 807-001) mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.1 (Artikelnr. 200 0191), geeignet für einen Viskositätsbereich bis 10.000 [mPa·s], Drehzahl Bereich 62,5 min-1, verwendet.

Werden in dieser Offenbarung Angaben zu einem Mittelwert gemacht, bezieht sich dieser Mittelwert immer auf arithmetische Mittel.

Werden in dieser Offenbarung Angaben zum Molekulargewicht von Polymeren gemacht, so beziehen diese sich auf eine Messung mittel Lichtstreuung.

Die Zubereitung der vorliegenden Erfindung umfasst ein erstes Guarhydroxypropyltrimethylammoniumchlorid. Dieses weist erfindungsgemäß ein mittleres Molekulargewicht von 2000000 bis 5000000 Da, bevorzugt 2000000 bis 3500000 Da und insbesondere bevorzugt von 2000000 bis 2500000 Da auf.

Weiterhin ist es erfindungsgemäß, wenn das erste Guarhydroxypropyltrimoniumchlorid dadurch gekennzeichnet ist, dass es eine Ladungsdichte von 0,5 bis 1,0 meq/g, bevorzugt von 0,6 bis 0,9 meq/g und insbesondere bevorzugt von 0,65 bis 0,85 meq/g.

Es ist weiterhin vorteilhaft, wenn der Anteil von dem ersten Guarhydroxypropyltrimoniumchlorid von 0,01 bis 0,5 Gew.-%, bevorzugt 0,03 bis 0,2 Gew.-% und insbesondere bevorzugt 0,06 bis 0,12 Gew.-%, bezogen auf das Gesamtgewicht der Zubereitung beträgt.

Die Zubereitung der vorliegenden Erfindung umfasst ein zweites Guar-Guarhydroxypropyltrimoniumchlorid. Dieses weist erfindungsgemäß ein mittleres Molekulargewicht von 400000 bis weniger als 2000000 Da, bevorzugt 600000 bis weniger als 1999999 Da und insbesondere bevorzugt von 1000000 bis 1900000 Da auf.

Weiterhin ist es erfindungsgemäß, wenn das zweite Guarhydroxypropyltrimoniumchlorid dadurch gekennzeichnet ist, dass es eine Ladungsdichte von 0,5 bis 1,0 meq/g, bevorzugt von 0,6 bis 0,9 meq/g und insbesondere bevorzugt von 0,65 bis 0,85 meq/g aufweist.

Es ist weiterhin vorteilhaft, wenn der Anteil von dem zweiten Guarhydroxypropyltrimoniumchlorid von 0,01 bis 0,5 Gew.-%, bevorzugt 0,03 bis 0,2 Gew.-% und insbesondere bevorzugt 0,06 bis 0,12 Gew.-%, bezogen auf das Gesamtgewicht der Zubereitung, beträgt.

Vorteilhaft beträgt der Gesamtanteil von dem ersten und zweiten Guarhydroxypropyltrimoniumchlorid von 0,02 bis 0,3 Gew.-%, bevorzugt von 0,07 bis 0,2 Gew.-% und insbesondere bevorzugt von 0,1 bis 0,19 Gew.-%, bezogen auf das Gesamtgewicht der Zubereitung.

Weiterhin ist es vorteilhaft im Sinne der vorliegenden Erfindung, wenn das Gewichtsverhältnis vom ersten zum zweiten Guarhydroxypropyltrimoniumchlorid von 2:1 bis 1:2, bevorzugt 1,5:1 bis 1:1,5 und insbesondere bevorzugt von 1,2:1 bis 1:1,2 beträgt.

Weiterhin ist es vorteilhaft, wenn die Zubereitung weitere kationische Polymere in Anteilen von weniger als 0,2 Gew.-%, bevorzugt weniger als 0,1 Gew.-% und insbesondere bevorzugt weniger als 0,05 Gew.-% und insbesondere bevorzugt von 0 Gew.-% enthält. Folglich ist es am bevorzugtesten, wenn keine weiteren kationischen Polymere enthalten sind.

Weiterhin ist es vorteilhaft, wenn die Zubereitung weitere Polymere, aus genommen von Alkylglycosiden, in Anteilen von weniger als 0,2 Gew.-%, bevorzugt weniger als 0,1 Gew.-% und insbesondere bevorzugt weniger als 0,05 Gew.-% und insbesondere bevorzugt von 0 Gew.-% enthält.

Die erfindungsgemäßen kosmetischen Zubereitungen enthalten Tenside. Vorteilhafte waschaktive anionische Tenside im Sinne der vorliegenden Erfindung sind Acylaminosäuren und deren Salze, wie
▪ Acylglutamate, insbesondere Natriumacylglutamat
▪ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
▪ Acylisethionate, z.B. Natrium-/ Ammoniumcocoylisethionat,
▪ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
sowie Schwefelsäureester, wie
▪ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
▪ Alkylsulfate, beispielsweise Natrium-, Ammonium- und TEA- Laurylsulfat.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.

Vorteilhafte waschaktive amphotere Tenside im Sinne der vorliegenden Erfindung sind
▪ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

Vorteilhafte waschaktive nicht-ionische Tenside im Sinne der vorliegenden Erfindung sind
▪ Alkanolamide, wie Cocamide MEA/ DEA/ MIPA,
▪ Ester, die durch Veresterung von Carbonsäuren mit Ethylenoxid, Glycerin, Sorbitan oder anderen Alkoholen entstehen,
▪ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
▪ Taurate, beispielsweise Natriumlauroyltaurat und Natriummethylcocoyltaurat,
▪ Ether-Carbonsäuren, beispielsweise Natriumlaureth-13 Carboxylat und Natrium PEG-6 Cocamide Carboxylat, Natrium PEG-7-Olivenöl-Carboxylat
▪ Phosphorsäureester und Salze, wie beispielsweise DEA-Oleth-10 Phosphat und Dilaureth-4 Phosphat,
▪ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat.

Weitere vorteilhafte amphotere Tenside sind
- N-Alkylaminosäuren, beispielsweise Aminopropylalkylglutamid, Alkylaminopropionsäure, Natriumalkylimidodipropionat und Lauroamphocarboxyglycinat und N-Kokosfettsäureamidoethyl-N-hydroxyethylglycinat Natriumsalze und deren Derivate.

Weitere vorteilhafte nicht-ionische Tenside sind Alkohole.

Weitere geeignete anionische Tenside im Sinne der vorliegenden Erfindung sind ferner
▪ Acylglutamate wie Di- TEA-palmitoylaspartat und Natrium Caprylic/ Capric Glutamat,
▪ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
▪ beispielsweise Laurinsäure, Aluminiumstearat, Magnesiumalkanolat und Zinkundecylenat,
▪ Ester-Carbonsäuren, beispielsweise Calciumstearoyllactylat, Laureth-6 Citrat und Natrium PEG-4 Lauramidcarboxylat,
▪ Alkylarylsulfonate.

Weitere geeignete kationische Tenside im Sinne der vorliegenden Erfindung sind ferner
▪ Alkylamine,
▪ Alkylimidazole,
▪ ethoxylierte Amine
insbesondere deren Salze.

Weitere geeignete nicht-ionische Tenside im Sinne der vorliegenden Erfindung sind ferner Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß besonders bevorzugt, wenn als Tenside anionische, amphotere und/oder nichtionische Tenside eingesetzt werden wobei es ganz besonders bevorzugt ist, wenn als Tenside Natriumlaurylethersulfat, Cocoamidopropylbetain und/oder Dinatrium PEG-5 Laurylcitratsulfosuccinat und/oder N-Kokosfettsäureamidoethyl-N-hydroxyethylglycinat Natriumsalze eingesetzt werden.

Ferner können Polysorbate als waschaktive Agentien erfindungsgemäß vorteilhaft in die Emulsion eingearbeitet werden.

Es ist erfindungsgemäß insbesondere vorteilhaft, wenn Natriumlaurylethersulfat als anionisches Tensid enthalten ist. Weiterhin ist es erfindungsgemäß insbesondere vorteilhaft, wenn Cocoamidopropylbetain als Tensid enthalten ist, insbesondere in Kombination mit Natriumlaurylethersulfat. Zusätzlich ist es vorteilhaft, wenn als Tensid mindestens ein Alkylglucosid, bevorzugt Decylglucoside enthalten ist. Das Decylglucosid wird vorteilhaft in Kombination mit Natriumlaurylethersulfat und Cocoamidopropylbetain eingesetzt.

Sofern Natriumlaurylethersulfat enthalten ist, beträgt der Anteil von Natriumlaurylethersulfat vorteilhaft von 4 bis 15 Gew.-%, bevorzugt von 6 bis 14 Gew.-% und insbesondere bevorzugt 7 bis 13 Gew.-%, bezogen auf das Gesamtgewicht der Zubereitung.

Sofern Cocoamidopropylbetain enthalten ist, beträgt der Anteil von Cocoamidopropylbetain vorteilhaft von 1 bis 10 Gew.-%, bevorzugt von 1,5 bis 8 Gew.-% und insbesondere bevorzugt 2 bis 6 Gew.-%, bezogen auf das Gesamtgewicht der Zubereitung.

Sofern ein Alkylglucosid, insbesondere Decylglucosid, enthalten ist, beträgt der Anteil von Alkylglucosid, insbesondere Decylglucosid, vorteilhaft von 0,1 bis 8 Gew.-%, bevorzugt von 0,5 bis 7 Gew.-% und insbesondere bevorzugt 0,75 bis 5 Gew.-%, bezogen auf das Gesamtgewicht der Zubereitung.

Es ist vorteilhaft im Sinne der vorliegenden Erfindung, wenn der Gesamtanteil der anionischen Tenside von 3 bis 18 Gew.-%, bevorzugt von 5 bis 15 Gew.-% und insbesondere bevorzugt 7 bis 14 Gew.-%, bezogen auf das Gesamtgewicht der Zubereitung, beträgt.

Es ist erfindungsgemäß von Vorteil, wenn ein oder mehrere Tenside in einer Konzentration von 1 bis 25 Gewichts-%, bevorzugt in einer Konzentration 7 von 20 bis Gewichts-% und ganz besonders bevorzugt in einer Konzentration von 8 bis 18 Gewichts-%, jeweils bezogen auf das Gesamtgewicht der Zubereitung eingesetzt werden.

Weiterhin ist es vorteilhaft, wenn die Zubereitung frei von Silikonverbindungen ist. Folglich beträgt der Anteil von Silikonverbindungen in der Zubereitung vorteilhaft von weniger als 2 Gew.-%, bevorzugt weniger als 1 Gew.-%, bevorzugt weniger als 0,5 Gew.-%, bevorzugt weniger als 0,2 Gew.-%, bevorzugt weniger als 0,1 Gew.-%, bevorzugt weniger als 0,05 Gew.-% und insbesondere bevorzugt 0 Gew.-%, bezogen auf das Gesamtgewicht der Zubereitung.

Weiterhin ist es vorteilhaft, wenn die Zubereitung frei von Polymeren aus Homo- oder Copolymerisation mit Acrylsäure und/oder Methacrylsäure ist. Folglich beträgt der Anteil von Polymere aus Homo- oder Copolymerisation mit Acrylsäure und/oder Methacrylsäure in der Zubereitung vorteilhaft von weniger als 2 Gew.-%, bevorzugt weniger als 1 Gew.-%, bevorzugt weniger als 0,5 Gew.-%, bevorzugt weniger als 0,2 Gew.-%, bevorzugt weniger als 0,1 Gew.-%, bevorzugt weniger als 0,05 Gew.-% und insbesondere bevorzugt 0 Gew.-%, bezogen auf das Gesamtgewicht der Zubereitung.

Weiterhin ist es vorteilhaft, wenn die Zubereitung frei von weiteren chemisch modifizierten katonischen Polymeren ist. Folglich beträgt der Anteil von weiteren chemisch modifizierten katonischen Polymeren in der Zubereitung vorteilhaft von weniger als 2 Gew.-%, bevorzugt weniger als 1 Gew.-%, bevorzugt weniger als 0,5 Gew.-%, bevorzugt weniger als 0,2 Gew.-%, bevorzugt weniger als 0,1 Gew.-%, bevorzugt weniger als 0,05 Gew.-% und insbesondere bevorzugt 0 Gew.-%, bezogen auf das Gesamtgewicht der Zubereitung.

Weiterhin ist es vorteilhaft, wenn die Zubereitung frei von Polyquaternium-10 ist. Folglich beträgt der Anteil von Polyquaternium-10 in der Zubereitung vorteilhaft von weniger als 2 Gew.-%, bevorzugt weniger als 1 Gew.-%, bevorzugt weniger als 0,5 Gew.-%, bevorzugt weniger als 0,2 Gew.-%, bevorzugt weniger als 0,1 Gew.-%, bevorzugt weniger als 0,05 Gew.-% und insbesondere bevorzugt 0 Gew.-%, bezogen auf das Gesamtgewicht der Zubereitung.

Weiterhin ist es vorteilhaft, wenn die Zubereitung frei von Substanzen, die eine PEG (Polyethylenglykol) - Untereinheit aufweisen, ist. Folglich beträgt der Anteil von Substanzen, die eine PEG (Polyethylenglykol) - Untereinheit aufweisen, in der Zubereitung vorteilhaft von weniger als 2 Gew.-%, bevorzugt weniger als 1 Gew.-%, bevorzugt weniger als 0,5 Gew.-%, bevorzugt weniger als 0,2 Gew.-%, bevorzugt weniger als 0,1 Gew.-%, bevorzugt weniger als 0,05 Gew.-% und insbesondere bevorzugt 0 Gew.-%, bezogen auf das Gesamtgewicht der Zubereitung.

Eine Polyethylenglykoluntereinheit besteht definitionsgemäß aus mindestens 2 aufeinanderfolgenden Ethylenglykoleinheiten.

Weiterhin ist es vorteilhaft, wenn die Zubereitung Sodium Benzoate umfasst. Sofern Sodium Benzoate enthalten ist, ist es weiterhin vorteilhaft, wenn der Anteil von Sodium Benzoate von 0,1 bis 0,8 Gew.-%, bevorzugt von 0,2 bis 0,7 Gew.-% und insbesondere bevorzugt von 0,3 bis 0,7 Gew.-%, bezogen auf das Gesamtgewicht der Zubereitung, beträgt.

Weiterhin ist es vorteilhaft, wenn die Zubereitung Natriumchlorid umfasst. Sofern Natriumchlorid enthalten ist, ist es weiterhin vorteilhaft, wenn der Anteil von Natriumchlorid von 0,1 bis 0,8 Gew.-%, bevorzugt von 0,2 bis 0,7 Gew.-% und insbesondere bevorzugt von 0,3 bis 0,7 Gew.-%, bezogen auf das Gesamtgewicht der Zubereitung, beträgt.

Weiterhin ist es vorteilhaft, wenn die Zubereitung Zitronensäure umfasst. Sofern Zitronensäure enthalten ist, ist es weiterhin vorteilhaft, wenn der Anteil von Zitronensäure von 0,3 bis 1,5 Gew.-%, bevorzugt von 0,5 bis 1,3 Gew.-% und insbesondere bevorzugt von 0,7 bis 1,0 Gew.-%, bezogen auf das Gesamtgewicht der Zubereitung, beträgt.

Weiterhin ist es vorteilhaft, wenn die Zubereitung Natriumcitrat umfasst. Sofern Natriumcitrat enthalten ist, ist es weiterhin vorteilhaft, wenn der Anteil von Natriumcitrat von 0,1 bis 0,8 Gew.-%, bevorzugt von 0,2 bis 0,7 Gew.-% und insbesondere bevorzugt von 0,3 bis 0,7 Gew.-%, bezogen auf das Gesamtgewicht des Konzentrats, beträgt.

Es ist ebenfalls vorteilhaft im Sinne der Erfindung, wenn die Zubereitung eine Mikroemulsion umfasst, welche 10 bis 20 Gew.% Alkylpolyglykoside, 4 bis 20 Gew.% Monoester des Glycerins von C12-C22 Fettsäuren und 5 bis 30 Gew.% Ölkörper aufweist, wobei sich die Angaben auf das Gesamtgewicht der Mikroemulsion beziehen.

Die Mikroemulsion enthält als zwingende Bestandteile ein Alkylpolyglykoside, und zwar ein Alkyl(oligo)glycosid (im Folgenden auch als "APG" bezeichnet). Alkyl- und/oder Alkenyloligoglycoside im Sinne der vorliegenden Lehre folgen dabei der Formel R¹O-[G]ₚ in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,5 liegt. APGs sind in den Mikroemulsionen gemäß der vorliegenden Erfindung in Mengen zwischen 10 und 20 Gew.-%, jeweils bezogen auf die Gesamtmenge der Mikroemulsion enthalten. Besonders bevorzugt sind dabei Mengen im Bereich von 14 bis 19 Gew.-%, bezogen auf die Mikroemulsion. Insbesondere bevorzugt wird Decyl Glucoside als Alkylpolyglykoside eingesetzt.

Weiterhin sind Monoester aus Fettsäuren der Kettenlänge C12-C22 mit Glycerin in den Emulsionen enthalten. Dabei sind insbesondere Monoester von Glycerin mit ungesättigten linearen Fettsäuren geeignet. Besonders bevorzugt im Sinne der Erfindung ist Glycerinmonooleat. Diese Glycerinester sind in den Mikroemulsionen in Mengen von 4 bis 20 Gew.-%, vorzugsweise 8 bis 15 Gew.-% - jeweils bezogen auf das Gesamtgewicht der Mikroemulsion - enthalten.

Schließlich enthält die Mikroemulsion noch einen Ölkörper, also eine nichtwasserlösliche organische Phase in Mengen von 5 bis 30 Gew.-%. Dabei sind besonders bevorzugte Ölphasen ausgewählt aus der Gruppe von Guerbetalkoholen auf Basis von Fettalkoholen mit 6 bis 18 C-Atomen, Estern linearer C6-C22-Fettsäuren mit linearen oder verzweigten C6-C22-Fettalkoholen bzw. Estern von verzweigten C6-C13-Carbonsäuren mit linearen oder verzweigten C6-C22-Fettalkoholen, Ester von linearen C6-C22-Fettsäuren mit verzweigten Alkoholen, Estern von C6-C22-Fettalkoholen und/oder Guerbetalkoholen mit aromatischen Carbonsäuren, Triglyceriden auf Basis C6-C10-Fettsäuren, flüssigen Mono-/Di-/Triglyceridmischungen auf Basis von C6-C18-Fettsäuren, Estern von C2-C12-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Öle, verzweigten primäre Alkoholen, substituierten Cyclohexanen, linearen und verzweigten C6-C22-Fettalkoholcarbonaten, Guerbetcarbonaten auf Basis von Fettalkoholen mit 6 bis 18, vorzugsweise 8 bis 10 C Atomen, Estern der Benzoesäure mit linearen und/oder verzweigten C6-C22-Alkoholen, linearen oder verzweigten, symmetrischen oder unsymmetrischen Dialkylethern mit 6 bis 22 Kohlenstoffatomen pro Alkylgruppe, aliphatischen bzw. naphthenischen Kohlenwasserstoffe und/oder Dialkylcyclohexanen. Als Ölkomponente können jedoch auch feste Fette und/oder Wachse. Diese können auch in Mischung mit den im vorherigen Abschnitt genannten Ölen vorliegen. Typische Beispiele für Fette sind Glyceride, d.h. feste oder flüssige pflanzliche oder tierische Produkte, die im Wesentlichen aus gemischten Glycerinestern höherer Fettsäuren bestehen. Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Tocopherole und ätherische Öle eignen sich ebenfalls als Ölkomponente. Insbesondere bevorzugt ist Dicaprylyl Ether als Ölkomponente.

Der Rest der Mikroemulsion umfasst auf 100 Gew.-% jeweils Wasser, wobei ggf ergänzende weitere Inhaltstoffe enthalten sein können.

EP2194956 B1 zeigt erfindungsgemäße Mikroemulsionen.

Der Anteil der Mikroemulsion in der Gesamtzubereitung beträgt vorteilhaft von 0,1 bis 2 Gew.-%, bevorzugt von 0,15 bis 0,5 Gew.-% und insbesondere bevorzugt von 0,2 bis 0,3 Gew.-%, bezogen auf das Gesamtgewicht der Zubereitung.

Es hat sich als besonders vorteilhaft herausgestellt, wenn die Komponenten der Mikroemulsion in Form einer Mikroemulsion zugegeben werden. Es ist weniger bevorzugt die Komponenten der Mikroemulsion ohne vorherige Emulgierung der Zubereitung zuzugeben.

Es ist vorteilhaft, wenn der Gesamtanteil von Polymeren, die von Alkylglucosiden verschieden sind, weniger als 0,3 Gew.-%, bevorzugt weniger als 0,25Gew.-% und insbesondere bevorzugt weniger als 0,2 Gew.-% beträgt.

Weiterhin ist es vorteilhaft, wenn die Zubereitung ein oder mehrere unter Normalbedingungen flüssige Parfümstoffe umfasst. Es ist bevorzugt, wenn der Anteil der flüssigen Parfümstoffe nicht mehr als 5 Gew.-% bezogen auf das Gesamtgewicht der Zubereitung, ausmacht. Vorteilhaft beträgt der Anteil der flüssigen Parfümstoffe von 0,01 bis 1,5 Gew.-%, bezogen auf das Gesamtgewicht der Zubereitung.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgende Tabelle zeigt mit Bsp.1 eine erfindungsgemäße Zubereitung, während Vgl.1 und Vgl.2 Vergleichsbeispiele darstellen. Vgl.1 zeigt ein konventionelles Shampoo, welches Polyquaternium-10 umfasst.

**Tab.2**

| **Inhaltstoffe** | **Bsp.1** | **Vgl.1** | **Vgl.2** |
|---|---|---|---|
| Aqua | Ad 100 | Ad 100 | Ad 100 |
| Cocamidopropyl Betaine | 3,36 | 3,36 | 3,50 |
| Sodium Chloride | 0,504 | 0,504 | 0,525 |
| Glycerin | 0,252 | 0,252 | 0,263 |
| Sodium Laureth Sulfate | 9,00 | 9,00 | 9,00 |
| PEG-40 Hydrogenated Castor Oil | | | 0,6 |
| Decyl Glucoside | 1,50 | 1,50 | |
| Polyquaternium-10 | | | 0,27 |
| Guar Hydroxypropyltrimonium Chloride* | | | 0,09 |
| Guar Hydroxypropyltrimonium Chloride** | 0,09 | | |
| Guar Hydroxypropyltrimonium Chloride*** | 0,09 | 0,18 | |
| Mikroemulsion aus 52,5% Aqua, 0,5% Benzoic Acid, 2% Citric Acid, 15% Decyl Glucoside; 20% Dicaprylyl Ether und 10% Glyceryl Oleate | 0,25 | 0,25 | |
| Sodium Citrate | 0,35 | 0,35 | |
| Citric Acid | 0,9 | 0,9 | 0,35 |
| Sodium Hydroxide | | | q.s. |
| Sodium Benzoate | 0,6 | 0,6 | 0,6 |
| Sodium Chloride | 1 | 1 | 1 |
| Parfum | 0,7 | 0,7 | 0,7 |

| | | | |
|---|---|---|---|
| *Kommerzielles Guarhydroxypropyltrimoniumchlorid; Jaguar^{®} Excel (Solvay) siehe Tabelle oben. **Kommerzielles Guarhydroxypropyltrimoniumchlorid aufweisend eine Masse im Bereich von mehr als 2,0 Millionen Dalton bis 2,5 Millionen Dalton und eine Ladungsdichte von 0,75 meq/g. ***Kommerzielles Guarhydroxypropyltrimoniumchlorid aufweisend eine Masse im Bereich von mehr als 1,2 Millionen Dalton bis weniger als 2,0 Millionen Dalton und eine Ladungsdichte von 0,72 meq/g. | | | |

Die vorstehenden Zubereitungen wurden in einem Konsumententest analysiert. An dem Konsumententest haben insgesamt 522 Personen teilgenommen. Dafür haben je 174 Konsumenten die Produkte Bsp.1, Vgl.1 oder Vgl.2 erhalten.

Die Konsumenten sollten das Produkt über einen Zeitraum von 10-12 Tage 3-4x pro Woche anwenden. Anschließend sollten sie die in Tab.3 aufgeführten Fragen beantworten. Die aufgeführten Messwerte stellen Mittelwerte der gegebenen Antworten dar.

**Tab.3**

| **Messergebnis** | **Bsp.1** | **Vgl.1** | **Vgl.2** |
|---|---|---|---|
| In wie weit fühlt sich der Schaum des Produktes beim Waschen der Haare cremig an (0 = nicht cremig und 100 = sehr cremig)? | 74 | 69 | 70 |
| Hinterlässt das Produkt ein angenehmes haptisches Gefühl während der Anwendung (0 = nicht angenehm und 100 = sehr angenehm)? | 82 | 72 | 74 |
| Wie leicht lässt sich das Produkt ausspülen (0 = sehr schlecht und 100 = sehr leicht)? | 88 | 78 | 86 |
| Fühlt sich das Haar weich beim Ausspülen des Produkts an? (0 = sehr rau und 100 = sehr weich)? | 79 | 69 | 73 |
| Wie beurteilen Sie die Reinigungsleistung? (0 = reinigt nicht gut und 100 reinigt sehr gut) | 80 | 65 | 71 |
| Wie ist das nasse Haar zu kämmen (0=schwer Kämmbar, hoher Widerstand und 100 besonders leicht Kämmbar, wenig Widerstand) | 69 | 66 | 55 |
| Wie ist das trockene Haar zu kämmen (0=schwer Kämmbar, hoher Widerstand und 100 besonders leicht Kämmbar, wenig Widerstand) | 67 | 62 | 59 |

Wie sich ergibt zeigt die erfindungsgemäße Shampoozubereitung des Bps.1 im Vergleich zu Vgl.1 und Vgl.2 deutlich verbessere Eigenschaften aufweist, ohne dass höhere Polymermengen eingesetzt werden müssen

Des Weiteren wurden die folgenden Zubereitungen bereitgestellt, wobei nur Bsp.2 ein erfindungsgemäßes Beispiel darstellt:

**Tab.4**

| **Inhaltsstoffe** | Bsp.2 | Vgl.3 | Vgl.4 | Vgl.5 |
|---|---|---|---|---|
| Aqua | Ad 100 | Ad 100 | Ad 100 | Ad 100 |
| Cocamidopropyl Betaine | 2,94 | 2,94 | 2,94 | 2,94 |
| Glycerin | 0,22 | 0,22 | 0,22 | 0,22 |
| Sodium Chloride | 0,44 | 0,44 | 0,44 | 0,44 |
| Sodium Laureth Sulfate | 9 | 9 | 9 | 9 |
| Mikroemulsion aus 52,5% Aqua, 0,5% Benzoic Acid, 2% Citric Acid, 15% Decyl Glucoside; 20% Dicaprylyl Ether und 10% Glyceryl Oleate | 0,25 | 0,25 | 0,25 | 0,25 |
| Decyl Glucoside | 1,50 | 1,50 | 1,50 | 1,50 |
| Polyquaternium-10 | | 0,18 | | |
| Guar Hydroxypropyltrimonium Chloride ** | 0,09 | | 0,18 | |
| Guar Hydroxypropyltrimonium Chloride *** | 0,09 | | | 0,18 |
| Citric Acid | 0,9 | 0,9 | 0,9 | 0,9 |
| Sodium Benzoate | 0,45 | 0,45 | 0,45 | 0,45 |
| Sodium Chloride | 0,5 | 0,5 | 0,5 | 0,5 |
| Parfume | 0,7 | 0,7 | 0,7 | 0,7 |
| | | | | |
| | | | | |

| **Messergebnisse:** | | | | |
|---|---|---|---|---|
| Gleitvermögen (30s) | 7,33 | 6,33 | 7 | 5,667 |
| Ausspülbarkeit (30s) | 5,67 | 5,33 | 5,333 | 5,333 |
| Kämmbarkeit | 6,67 | 5,67 | 5,667 | 6 |

| | | | | |
|---|---|---|---|---|
| ** und *** siehe oben. | | | | |

Die Zubereitungen gezeigt in Tab.4 wurden in einer weiteren Panelstudie mit 5 Teilnehmern, welche geschultes Personal sind, untersucht. Die 5 Teilnehmer haben zur Beurteilung der Parameter Echthaartressen gewaschen. Dazu wurde das folgende Protokoll durchgeführt:
Eine Echthaarstresse (10g freies Haargewicht, 0,5h gebleicht) wird mit 0,2g Produkt / 1g Haar behandelt. Beim Aufschäumen des Produktes wird nach 30 sec das Gleitvermögen beurteilt (Ein Wert von 0 bedeutet, dass es schwierig ist, über die Tresse zu gleiten, da der Widerstand zu groß ist und ein Wert von 10 bedeutet, dass man ohne Widerstand über die Tresse gleiten kann). Danach wird das Produkt mit handwarmem Leitungswasser ausgespült und dabei nach 30 sec die Ausspülbarkeit bewertet (0 = schlecht ausspülbar, 10 leicht ausspülbar). Nach dem Ausspülen des Produktes wird die Nasskämmbarkeit mittels Kamm (Fa. Hercules Sägemann, No. 623.7/394.7, feine Seite) beurteilt (0=schwer Kämmbar, hoher Widerstand und 10 besonders leicht Kämmbar, wenig Widerstand)

Die Ergebnisse sind ebenfalls in Tab.4 angegeben. Vergleicht man die Ergebnisse zu Vgl.2 so zeigt sich wiederum, dass trotz geringerer Polymerkonzentration und Verzicht auf Polyquaternium-10 eine deutlich verbessertes Gleitvermögen, Ausspülbarkeit und Kämmbarkeit erzielt wird. Ein weiterer Vergleich von Bsp.2 zu Vgl.3 und Vgl.4 zeigt, dass in Hinblick auf alle Parameter eine synergistische Verbesserung der Parameter gegenüber dem Einzeleinsatz des Guarhydroxypropyltrimoniumchlorids erzielt wird.

Weitere Beispiele sind in der folgenden Tabelle gezeigt:

| **Inhaltstoffe** | **Bsp.1** | **Bsp. 2** | **Bsp. 3** | **Bsp. 4** | **Bsp. 5** | **Bsp. 6** |
|---|---|---|---|---|---|---|
| Aqua | Ad 100 | Ad 100 | Ad 100 | Ad 100 | Ad 100 | Ad 100 |
| Cocamidopropyl Betaine | 3,36 | 3,36 | 2,94 | 3,50 | 3,36 | 3,20 |
| Sodium Chloride | 0,504 | 0,504 | 0,44 | 0,53 | 0,504 | 0,48 |
| Glycerin | 0,252 | 0,252 | 0,22 | 0,26 | 0,252 | 0,24 |
| Sodium Laureth Sulfate | 9 | 8 | 9 | 10 | 8 | 9 |
| Decyl Glucoside | 1,50 | 2,00 | 2,00 | 1,00 | 2,00 | 1,50 |
| Guar Hydroxypropyltrimonium Chloride** | 0,09 | 0,09 | 0,09 | 0,09 | 0,135 | 0,09 |
| Guar Hydroxypropyltrimonium Chloride*** | 0,09 | 0,18 | 0,18 | 0,09 | 0,135 | 0,18 |
| Mikroemulsion aus 52,5% Aqua, 0,5% Benzoic Acid, 2% Citric Acid, 15% Decyl Glucoside; 20% Dicaprylyl Ether und 10% Glyceryl Oleate | 0,25 | 0,25 | 0,75 | 0,50 | 0,25 | 1,00 |
| Sodium Citrate | 0,35 | 0,35 | | 0,35 | | |
| Citric Acid | 0,9 | 0,8 | 0,75 | 0,9 | 0,7 | 0,75 |
| Sodium Benzoate | 0,6 | 0,6 | 0,6 | 0,6 | 0,6 | 0,6 |
| Sodium Chloride | 0,7 | 0,8 | 0,7 | 0,6 | 0,8 | 0,7 |
| Parfum | 0,7 | 0,6 | 0,5 | 0,5 | 0,7 | 0,7 |

## Patentansprüche

1. Shampoozubereitung enthaltend
a. mindestens ein erstes Guarhydroxypropyltrimoniumchlorid, welches ein mittleres Molekulargewicht von mehr als 2000000 bis 5000000 Dalton aufweist und eine Ladungsdichte von 0,5 bis 1,0 meq/g besitzt,
b. mindestens ein zweites Guarhydroxypropyltrimoniumchlorid, welches ein mittleres Molekulargewicht von 400000 bis weniger als 2000000 Dalton aufweist und eine Ladungsdichte von 0,5 bis 1,0 meq/g besitzt,
c. ein oder mehrere Tenside
wobei das mittlere Molekulargewicht mittels Lichtstreuung ermittelt wird.

2. Shampoozubereitung nach Anspruch 1 **dadurch gekennzeichnet, dass** das erste Guarhydroxypropyltrimoniumchlorid ein mittleres Molekulargewicht von 2000000 bis 3500000 Da und bevorzugt von 2000000 bis 2500000 Da aufweist.

3. Shampoozubereitung nach einem der vorhergehenden Ansprüche **dadurch gekennzeichnet, dass** das erste Guarhydroxypropyltrimoniumchlorid **dadurch gekennzeichnet ist, dass** es eine Ladungsdichte von 0,6 bis 0,9 meq/g und bevorzugt von 0,65 bis 0,85 meq/g beträgt.

4. Shampoozubereitung nach einem der vorhergehenden Ansprüche **dadurch gekennzeichnet, dass** der Anteil von dem ersten Guarhydroxypropyl-trimonium-chlorid von 0,01 bis 0,5 Gew.-%, bevorzugt 0,03 bis 0,2 Gew.-% und insbesondere bevorzugt 0,06 bis 0,12 Gew.-%, bezogen auf das Gesamtgewicht der Zubereitung beträgt.

5. Shampoozubereitung nach einem der vorhergehenden Ansprüche **dadurch gekennzeichnet, dass** das zweite Guarhydroxypropyltrimoniumchlorid ein mittleres Molekulargewicht von 600000 bis weniger als 1999999 Da und bevorzugt von 1000000 bis 1900000 Da aufweist.

6. Shampoozubereitung nach einem der vorhergehenden Ansprüche **dadurch gekennzeichnet, dass** das zweite Guarhydroxypropyltrimoniumchlorid **dadurch gekennzeichnet ist, dass** es eine Ladungsdichte von 0,6 bis 0,9 meq/g und bevorzugt von 0,65 bis 0,85 meq/g aufweist.

7. Shampoozubereitung nach einem der vorhergehenden Ansprüche **dadurch gekennzeichnet, dass** der Anteil von dem zweiten Guarhydroxypropyltrimoniumchlorid von 0,01 bis 0,5 Gew.-%, bevorzugt 0,03 bis 0,2 Gew.-% und insbesondere bevorzugt 0,06 bis 0,12 Gew.-%, bezogen auf das Gesamtgewicht der Zubereitung, beträgt.

8. Shampoozubereitung nach einem der vorhergehenden Ansprüche **dadurch gekennzeichnet, dass** das Gewichtsverhältnis vom ersten zum zweiten Guarhydroxypropyltrimoniumchlorid von 2:1 bis 1:2, bevorzugt 1,5:1 bis 1:1,5 und insbesondere bevorzugt von 1,2:1 bis 1:1,2 beträgt.

9. Shampoozubereitung nach einem der vorhergehenden Ansprüche **dadurch gekennzeichnet, dass** die Zubereitung weitere kationische Polymere in Anteilen von weniger als 0,2 Gew.-%, bevorzugt weniger als 0,1 Gew.-% und insbesondere bevorzugt weniger als 0,05 Gew.-% und insbesondere bevorzugt von 0 Gew.-% enthält.

10. Shampoozubereitung nach einem der vorhergehenden Ansprüche **dadurch gekennzeichnet, dass** ein oder mehrere Tenside in einer Konzentration von 1 bis 25 Gewichts-%, bevorzugt in einer Konzentration 7 von 20 bis Gewichts-% und ganz besonders bevorzugt in einer Konzentration von 8 bis 18 Gewichts-%, jeweils bezogen auf das Gesamtgewicht der Zubereitung eingesetzt werden.

11. Shampoozubereitung nach einem der vorhergehenden Ansprüche **dadurch gekennzeichnet, dass** anionische Tenside enthalten sind, wobei Gesamtanteil der anionischen Tenside von 3 bis 18 Gew.-%, bevorzugt von 5 bis 15 Gew.-% und insbesondere bevorzugt 7 bis 14 Gew.-%, bezogen auf das Gesamtgewicht der Zubereitung, beträgt.

12. Shampoozubereitung nach einem der vorhergehenden Ansprüche **dadurch gekennzeichnet, dass** Cocoamidopropylbetain als Tensid enthalten ist, wobei der Anteil von Cocoamidopropylbetain vorteilhaft von 1 bis 10 Gew.-%, bevorzugt von 1,5 bis 8 Gew.-% und insbesondere bevorzugt 2 bis 6 Gew.-%, bezogen auf das Gesamtgewicht der Zubereitung, beträgt.

13. Shampoozubereitung nach einem der vorhergehenden Ansprüche **dadurch gekennzeichnet, dass** als Tensid ein Alkylglucosid, insbesondere Decylglucosid, enthalten ist, wobei der Anteil von Alkylglucosid, insbesondere Decylglucosid, vorteilhaft von 0,1 bis 8 Gew.-%, bevorzugt von 0,5 bis 7 Gew.-% und insbesondere bevorzugt 0,75 bis 5 Gew.-%, bezogen auf das Gesamtgewicht der Zubereitung, beträgt.

14. Shampoozubereitung nach einem der vorhergehenden Ansprüche **dadurch gekennzeichnet, dass** die Zubereitung frei von Polymeren aus Homo- oder Copolymerisation mit Acrylsäure und/oder Methacrylsäure ist.

15. Shampoozubereitung nach einem der vorhergehenden Ansprüche **dadurch gekennzeichnet, dass** die Zubereitung eine Mikroemulsion umfasst, welche 10 bis 20 Gew.% Alkylpolyglykoside, 4 bis 20 Gew.% Monoester des Glycerins von C12-C22 Fettsäuren und 5 bis 30 Gew.% Ölkörper aufweist, wobei sich die Angaben auf das Gesamtgewicht der Mikroemulsion beziehen.

16. Shampoozubereitung nach einem der vorhergehenden Ansprüche **dadurch gekennzeichnet, dass** der Gesamtanteil von Polymeren, die von Alkylglucosiden verschieden sind, weniger als 0,3 Gew.-%, bevorzugt weniger als 0,25Gew.-% und insbesondere bevorzugt weniger als 0,2 Gew.-% beträgt, bezogen auf das Gesamtgewicht der Zubereitung.

## Claims

1. Shampoo preparation comprising
a. at least one first guar hydroxypropyltrimonium chloride which has an average molecular weight of more than 2 000 000 to 5 000 000 daltons and a charge density of 0.5 to 1.0 meq/g,
b. at least one second guar hydroxypropyltrimonium chloride which has an average molecular weight of 400 000 to less than 2 000 000 daltons and a charge density of 0.5 to 1.0 meq/g,
c. one or more surfactants,
where the average molecular weight is determined by light scattering.

2. Shampoo preparation according to Claim 1, **characterized in that** the first guar hydroxypropyltrimonium chloride has an average molecular weight of 2 000 000 to 3 500 000 Da and preferably of 2 000 000 to 2 500 000 Da.

3. Shampoo preparation according to either of the preceding claims, **characterized in that** the first guar hydroxypropyltrimonium chloride is **characterized in that** it has a charge density of 0.6 to 0.9 meq/g and preferably of 0.65 to 0.85 meq/g.

4. Shampoo preparation according to any of the preceding claims, **characterized in that** the proportion of the first guar hydroxypropyltrimonium chloride is from 0.01% to 0.5% by weight, preferably 0.03% to 0.2% by weight and especially preferably 0.06% to 0.12% by weight, based on the total weight of the preparation.

5. Shampoo preparation according to any of the preceding claims, **characterized in that** the second guar hydroxypropyltrimonium chloride has an average molecular weight of 600 000 to less than 1 999 999 Da and preferably of 1 000 000 to 1 900 000 Da.

6. Shampoo preparation according to any of the preceding claims, **characterized in that** the second guar hydroxypropyltrimonium chloride is **characterized in that** it has a charge density of 0.6 to 0.9 meq/g and preferably of 0.65 to 0.85 meq/g.

7. Shampoo preparation according to any of the preceding claims, **characterized in that** the proportion of the second guar hydroxypropyltrimonium chloride is from 0.01% to 0.5% by weight, preferably 0.03% to 0.2% by weight and especially preferably 0.06% to 0.12% by weight, based on the total weight of the preparation.

8. Shampoo preparation according to any of the preceding claims, **characterized in that** the weight ratio of the first to the second guar hydroxypropyltrimonium chloride is from 2:1 to 1:2, preferably 1.5:1 to 1:1.5 and especially preferably from 1.2:1 to 1:1.2.

9. Shampoo preparation according to any of the preceding claims, **characterized in that** the preparation comprises further cationic polymers in proportions of less than 0.2% by weight, preferably less than 0.1% by weight and especially preferably less than 0.05% by weight and especially preferably of 0% by weight.

10. Shampoo preparation according to any of the preceding claims, **characterized in that** one or more surfactants are used in a concentration of 1% to 25% by weight, preferably in a concentration of 7% to 20% by weight and very particularly preferably in a concentration of 8% to 18% by weight, in each case based on the total weight of the preparation.

11. Shampoo preparation according to any of the preceding claims, **characterized in that** anionic surfactants are present, where the total proportion of the anionic surfactants is from 3% to 18% by weight, preferably from 5% to 15% by weight and especially preferably 7% to 14% by weight, based on the total weight of the preparation.

12. Shampoo preparation according to any of the preceding claims, **characterized in that** cocamidopropyl betaine is present as surfactant, where the proportion of cocamidopropyl betaine is advantageously from 1% to 10% by weight, preferably from 1.5% to 8% by weight and especially preferably 2% to 6% by weight, based on the total weight of the preparation.

13. Shampoo preparation according to any of the preceding claims, **characterized in that** an alkyl glucoside, in particular decyl glucoside, is present as surfactant, where the proportion of alkyl glucoside, in particular decyl glucoside, is advantageously from 0.1% to 8% by weight, preferably from 0.5% to 7% by weight and especially preferably 0.75% to 5% by weight, based on the total weight of the preparation.

14. Shampoo preparation according to any of the preceding claims, **characterized in that** the preparation is free of polymers from homo- or copolymerization with acrylic acid and/or methacrylic acid.

15. Shampoo preparation according to any of the preceding claims, **characterized in that** the preparation comprises a microemulsion which comprises 10% to 20% by weight of alkyl polyglycosides, 4% to 20% by weight of glycerol monoesters of C12-C22 fatty acids and 5% to 30% by weight of oil bodies, where the figures are based on the total weight of the microemulsion.

16. Shampoo preparation according to any of the preceding claims, **characterized in that** the total proportion of polymers which are different from alkyl glucosides is less than 0.3% by weight, preferably less than 0.25% by weight and especially preferably less than 0.2% by weight, based on the total weight of the preparation.

## Revendications

1. Préparation de shampooing contenant
a. au moins un premier chlorure d'hydroxypropyltrimonium de guar, qui présente une masse moléculaire moyenne de plus de 2 000 000 à 5 000 000 daltons et possède une densité de charge de 0,5 à 1,0 méq/g,
b. au moins un deuxième chlorure d'hydroxypropyltrimonium de guar, qui présente une masse moléculaire moyenne de 400 000 à moins de 2 000 000 daltons et possède une densité de charge de 0,5 à 1,0 méq/g,
c. un ou plusieurs tensioactifs
la masse moléculaire moyenne étant déterminée par diffusion de lumière.

2. Préparation de shampooing selon la revendication 1, **caractérisée en ce que** le premier chlorure d'hydroxypropyltrimonium de guar présente une masse moléculaire moyene de 2 000 000 à 3 500 000 Da, de préférence de 2 000 000 à 2 500 000 Da.

3. Préparation de shampooing selon l'une quelconque des revendications précédentes, **caractérisée en ce que** le premier chlorure d'hydroxypropyltrimonium de guar est **caractérisé en ce qu'**il présente une densité de charge de 0,6 à 0,9 méq/g, de préférence de 0,65 à 0,85 méq/g.

4. Préparation de shampooing selon l'une quelconque des revendications précédentes, **caractérisée en ce que** la proportion du premier chlorure d'hydroxypropyltrimonium de guar représente 0,01 à 0,5 % en poids, de préférence 0,03 à 0,2 % en poids et de manière particulièrement préférée 0,06 à 0,12 % en poids, par rapport au poids total de la préparation.

5. Préparation de shampooing selon l'une quelconque des revendications précédentes, **caractérisée en ce que** le deuxième chlorure d'hydroxypropyltrimonium de guar présente une masse moléculaire moyenne de 600 000 à moins de 1 999 999 Da, de préférence de 1 000 000 à 1 900 000 Da.

6. Préparation de shampooing selon l'une quelconque des revendications précédentes, **caractérisée en ce que** le deuxième chlorure d'hydroxypropyltrimonium de guar est **caractérisé en ce qu'**il présente une densité de charge de 0,6 à 0,9 méq/g, de préférence de 0,65 à 0,85 méq/g.

7. Préparation de shampooing selon l'une quelconque des revendications précédentes, **caractérisée en ce que** la proportion du deuxième chlorure d'hydroxypropyltrimonium de guar représente 0,01 à 0,5 % en poids, de préférence 0,03 à 0,2 % en poids et de manière particulièrement préférée 0,06 à 0,12 % en poids, par rapport au poids total de la préparation.

8. Préparation de shampooing selon l'une quelconque des revendications précédentes, **caractérisée en ce que** le rapport pondéral du premier au deuxième chlorure d'hydroxypropyltrimonium de guar est de 2:1 à 1:2, de préférence de 1,5:1 à 1:1,5, et de manière particulièrement préférée de 1,2:1 à 1:1,2.

9. Préparation de shampooing selon l'une quelconque des revendications précédentes, **caractérisée en ce que** la préparation contient d'autres polymères cationiques dans des proportions inférieures à 0,2 % en poids, de préférence inférieures à 0,1 % en poids, de manière particulièrement préférée inférieures à 0,05 % en poids, de manière particulièrement préférée de 0 % en poids.

10. Préparation de shampooing selon l'une quelconque des revendications précédentes, **caractérisée en ce que** l'on utilise un ou plusieurs tensioactifs à une concentration de 1 à 25 % en poids, de préférence à une concentration de 7 à 20 % en poids et tout particulièrement de préférence à une concentration de 8 à 18 % en poids, chaque fois par rapport au poids total de la préparation.

11. Préparation de shampooing selon l'une quelconque des revendications précédentes, **caractérisée en ce que** des tensioactifs anioniques sont contenus, la proportion totale des tensioactifs anioniques étant de 3 à 18 % en poids, de préférence de 5 à 15 % en poids et de manière particulièrement préférée de 7 à 14 % en poids, par rapport au poids total de la préparation.

12. Préparation de shampoing selon l'une quelconque des revendications précédentes, **caractérisée en ce que** de la cocoamidopropylbétaïne est contenue en tant que tensioactif, la proportion de cocoamidopropylbétaïne valant de 1 à 10 % en poids, de préférence de 1,5 à 8 % en poids, et de manière particulièrement préférée de 2 à 6 % en poids, par rapport au poids total de la préparation.

13. Préparation de shampooing selon l'une quelconque des revendications précédentes, **caractérisée en ce qu'**elle contient comme tensioactif un alkylglucoside, en particulier un décylglucoside, la proportion d'alkylglucoside, en particulier de décylglucoside, étant avantageusement de 0,1 à 8 % en poids, de préférence de 0,5 à 7 % en poids, de manière particulièrement préférée de 0,75 à 5 % en poids, par rapport au poids total de la préparation.

14. Préparation de shampooing selon l'une quelconque des revendications précédentes, **caractérisée en ce que** la préparation est exempte de polymères provenant d'homo- ou copolymérisation avec l'acide acrylique et/ou l'acide méthacrylique.

15. Préparation de shampooing selon l'une quelconque des revendications précédentes, **caractérisée en ce que** la préparation comprend une microémulsion comprenant 10 à 20 % en poids d'alkylpolyglycosides, 4 à 20 % en poids de monoesters de glycérol d'acides gras en C12-C22 et de 5 à 30 % en poids de corps huileux, les chiffres se rapportant au poids total de la microémulsion.

16. Préparation de shampooing selon l'une quelconque des revendications précédentes, **caractérisée en ce que** la proportion totale de polymères autres que les alkylglucosides est inférieure à 0,3 % en poids, de préférence inférieure à 0,25 % en poids et de manière particulièrement préférée inférieure à 0,2 % en poids, par rapport au poids total de la préparation.
